# EUROPEAN PATENT APPLICATION

(11) **EP 3 918 977 A1**
(43) Date of publication of application: **08.12.2021**
(21) Application number: 19912866.1
(22) Date of filing: 19.06.2019
(51) Int. Cl.: A61B 5/00, A61B 5/15

(54) **MINIMALLY INVASIVE SKIN BIOPSY METHOD USING MICRONEEDLE PATCH**

(30) Priority: 30.01.2019 KR 20190011763
(71) Applicant: Raphas Co., Ltd., Seoul 07793 (KR)
(72) Inventor: LEE, Keun Ho, Goyang-si, Gyeonggi-do 10508 (KR); AN, Eun Jin, Seoul 07803 (KR); KIM, Seong Jin, Seoul 07788 (KR); SUNG, Chang Yub, Bucheon-si, Gyeonggi-do 14589 (KR); KIM, Jung Dong, Incheon 22704 (KR); JEONG, Do Hyeon, Seoul 03904 (KR); LEE, Kwang Hoon, Seoul 07333 (KR)
(74) Representative: RGTH
(86) International application number: PCT/KR2019/007413
(87) International publication number: WO 2020/159013

(57) **Abstract**

The present disclosure relates to a minimally-invasive skin biopsy method. The minimally-invasive skin biopsy method according to the present disclosure includes: preparing a microneedle patch; attaching the microneedle patch to a skin of an object; maintaining a state in which the microneedle patch is attached to the skin of the object for a predetermined period of time; and detaching the microneedle patch from the skin of the object after the predetermined period of time, and analyzing a protein component of the object, which adheres to a microneedle of the microneedle patch. The microneedle is made of a biocompatible polymer substance.

## Description

### TECHNICAL FIELD

The present disclosure relates to a minimally-invasive skin biopsy method using a microneedle patch.

### BACKGROUND

Although a great number of drugs and bioactive substances for treating diseases has been developed, it is challenging to deliver the drugs and bioactive substances into the body due to a biological barrier (for example, skin, oral mucosa, brain-blood barrier and the like) and thus, problems regarding passing through the biological barrier and delivery efficiency of drugs still remain.

In general, drugs and bioactive substances are orally administered in the form of tablets or capsules. However, various drugs are digested or absorbed in the gastrointestinal tract or lost due to a liver mechanism and accordingly, the drugs and bioactive substances may not be efficiently delivered. In addition, several drugs pass through intestinal mucous membrane and may not be effectively spread. Patient's compliance is also an issue (for example, if a patient needs to take medicine in a regular interval, or critical patents who cannot take medicine).

Other general way of delivering drugs and bioactive substances includes using conventional needles. It is a more efficient way than oral administration, however, may cause pain on injection sites, a local damage on skin, bleeding, and disease infection on injection sites.

In order to solve problems of oral administration and subcutaneous injection, transdermal administration using patches are used. In the transdermal administration using patches, patient compliance is high, and drug level in blood remains steady.

Various microstructures including microneedles as one of the transdermal administration described above have been developed. Various metallic materials and various polymeric substances are used as the material of the microneedles. In recent years, a biodegradable polymer substance has attracted attention as the material of the microneedles.

A representative method of fabricating a microstructure made of a biodegradable substance is one using a mold. The microstructure, namely the microneedles, is formed by initially fabricating a mold having intaglio corresponding to the shape of the microstructure to be formed through the application of a semiconductor manufacturing process, pouring a material of the microstructure into the mold, and separating the material of the microstructure from the mold after the material is coagulated.

The present applicant has a number of domestic and foreign patents related to a microstructure fabricating method using a droplet air-born blowing manner, as a new manufacturing method that can be used instead of the microstructure fabricating method using a mold. The droplet air-born blowing manner will be described in brief below. The droplet air-born blowing manner includes: spotting a biocompatible polymer substance having viscosity on a bottom layer of a patch placed on a substrate or the bottom layer of the patch before being coupled to the patch; spotting the same biocompatible polymer substance on a bottom layer of a patch placed on another substrate or the bottom layer of the patch before being coupled to the patch (or omitting this step); inverting the another substrate upside down; approaching the inverted substrate toward the spotted biocompatible polymer substance so that the inverted substrate is brought into contact with the biocompatible polymer substance; stretching the biocompatible polymer substance having viscosity which has been brought into contact with the two substrates while keeping a relative distance between the two substrates; performing air blowing after the stretching process to fix the stretched state of the biocompatible polymer substance; cutting a middle portion of the stretched biocompatible polymer substance so that the same microstructure is formed on each of two different substrates.

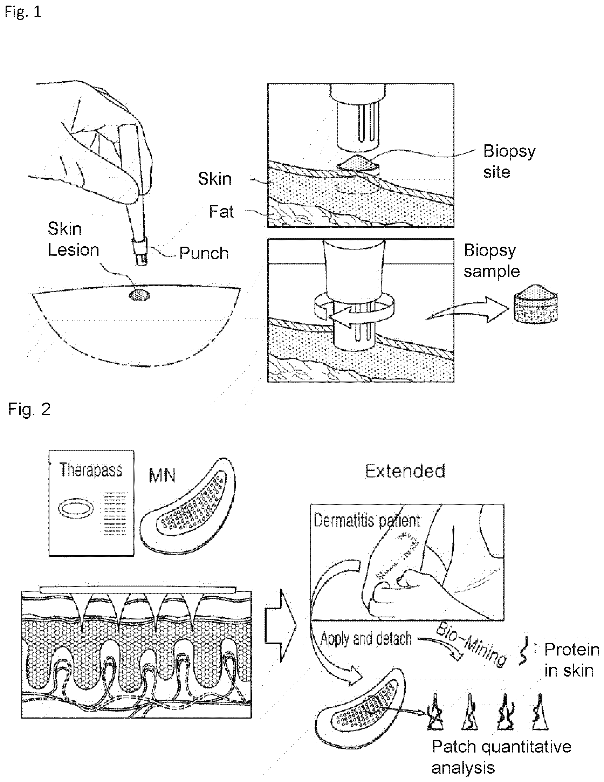

In the above, the technical meaning of the microstructure, which is a means for delivering drugs and bioactive substances, the method of fabricating the microstructure, and the like have been described in brief. The present inventors have suggested that the microstructure of the present disclosure is utilized for a new purpose rather than the purpose of the delivery of drugs and bioactive substances. As an example, a patch provided with microneedles, namely a microstructure, may be used for a minimally-invasive skin biopsy. FIG. 1 shows a process of a conventional skin biopsy. As shown in FIG. 1, the conventional skin biopsy may acquire a protein tissue in the skin in a reliable manner and provide the same for inspection. However, this process is very invasive, causes pain to the patient, and has disadvantages such as bleeding and scarring on the skin of the patient.

Another conventional skin biopsy includes a taping technique. The article titled "Measurements of AMPs in stratum corneum of atopic dermatitis and healthy skin-tape stripping technique" in SCIENTIFIC REPORTS, January 16, 2018 discloses a skin biopsy method which includes attaching a viscous tape to the skin, detaching the tape from the skin after a predetermined period of time, and examining a skin tissue adhered to the tape.

In addition, the article titled "Stratum Corneum Tape Stripping: Monitoring of Inflammatory Mediators in Atopic Dermatitis Patients Using Topical Therapy" in Allergy and Immunology, September 2, 2016, discloses a method using a viscous tape in skin biopsy for an atopic dermatitis patient.

This taping method has advantages of not causing any problems such as bleeding or scarring because it is simple and not invasive. However, this method has a very low reliability compared to the biopsy method illustrated in FIG. 1 from the viewpoint of acquiring a protein tissue for biopsy.

The present inventors have continued study for the purpose of providing a simplified skin biopsy method without causing pain to a patient in a minimally-invasive manner while acquiring a skin protein sufficient to provide reliable examination results by solving the disadvantages of the two skin biopsy methods described above, and completed the present disclosure.

### SUMMARY

An object of the present disclosure is to provide a minimally-invasive skin biopsy method using a microneedle patch in a simplified manner, which is capable of acquiring a sufficient amount of skin proteins to provide a reliable examination result without causing pain to a patient in a minimally-invasive manner.

A minimally-invasive skin biopsy method according to an embodiment of the present disclosure includes: preparing a microneedle patch; attaching the microneedle patch to a skin of an object; maintaining a state in which the microneedle patch is attached to the skin of the object for a predetermined period of time; and detaching the microneedle patch from the skin of the object after the predetermined period of time, and analyzing a protein component of the object, which adhered to a microneedle of the microneedle patch. A microneedle is made of a biocompatible polymer substance.

The biocompatible polymer substance, which is a material of the microneedle, may be a hyaluronic acid or a chitosan.

When the material of the microneedle is the hyaluronic acid, the predetermined period of time may be preferably determined in advance in consideration of a dissolution rate according to a molecular weight of the hyaluronic acid.

As the molecular weight of the hyaluronic acid increases, the predetermined period of time for which the microneedle patch is attached to the skin of the object may be increased to improve a biopsy performance.

Furthermore, additional features may further be provided to the method according to the present disclosure.

According to the present disclosure, it is possible to provide a method of performing a minimally-invasive skin biopsy using a microneedle patch in a simplified manner, which is capable of acquiring a sufficient amount of skin proteins to provide a reliable examination result without causing pain to a patient in a minimally-invasive manner.

### BRIEF DESCRIPTION OF THE DRAWINGS

FIG. 1 is a view illustrating a conventional skin biopsy method.
FIG. 2 is a conceptual view illustrating a skin biopsy method according to the present disclosure.

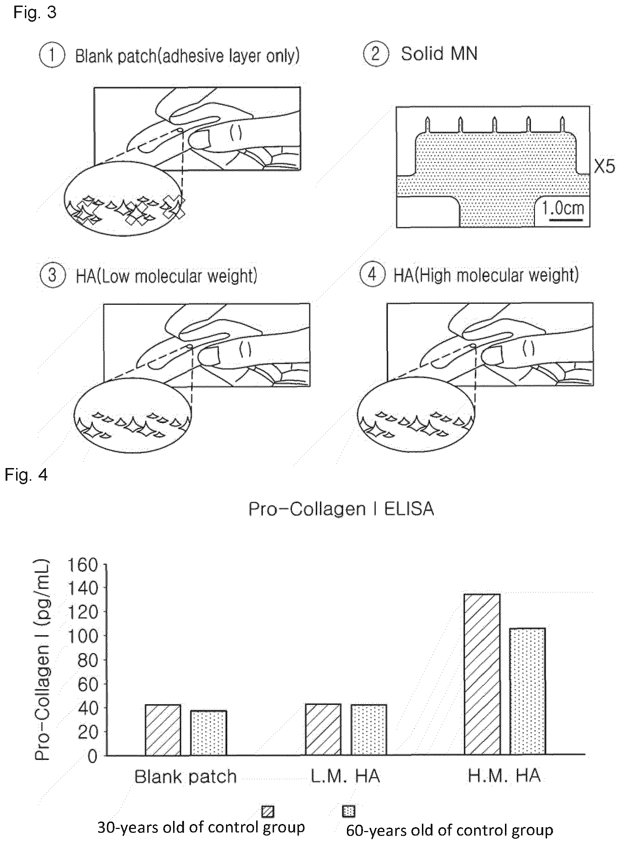

FIG. 3 is a view showing four control groups to be tested in a first embodiment of the present disclosure, wherein the first control group (1) is a blank patch not provided with microneedles, the second control group (2) is a solid microneedle made of a metallic material, the third control group (3) is a microneedle patch formed of low-molecular-weight hyaluronic acid, and the fourth control group (4) is a microneedle patch formed of high-molecular weight hyaluronic acid.
FIG. 4 is a chart showing results obtained by performing a Pro-Collagen 1 detection experiment with respect to each of a 30-years old of patient group and a 60-years old of patient group with the four control groups used in the first embodiment of the present disclosure.

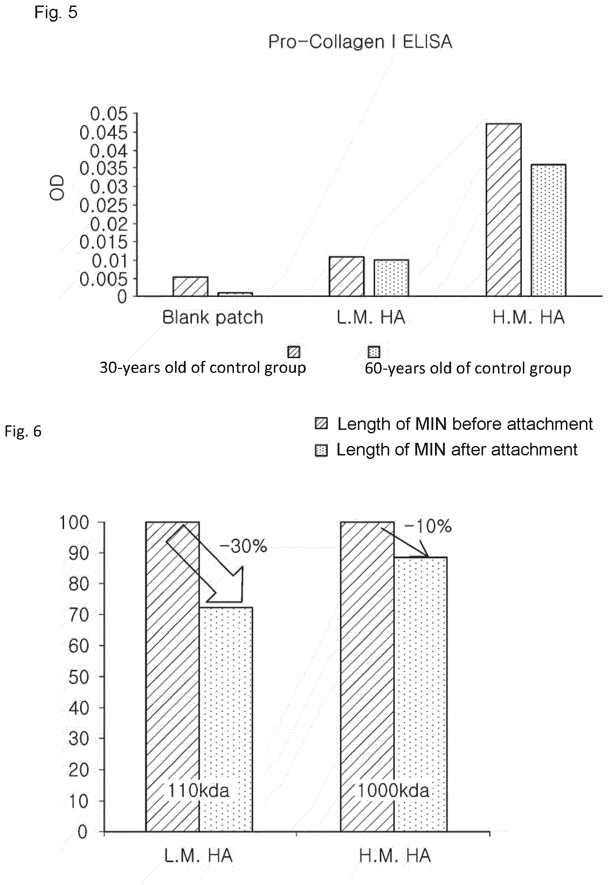

FIG. 5 is a chart showing results obtained by performing the Pro-Collagen 1 detection experiment with respect to each of a 30-years old of patient group and a 60-years old of patient group with the four control groups used in the first embodiment of the present disclosure, in which the y axis represents absorbance.
FIG. 6 is a chart showing results obtained by measuring changes in length before and after performing skin biopsy using a microneedle patch formed of low-molecular-weight hyaluronic acid and a microneedle patch formed of high-molecular-weight hyaluronic acid among the four control groups used in the first embodiment of the present disclosure.

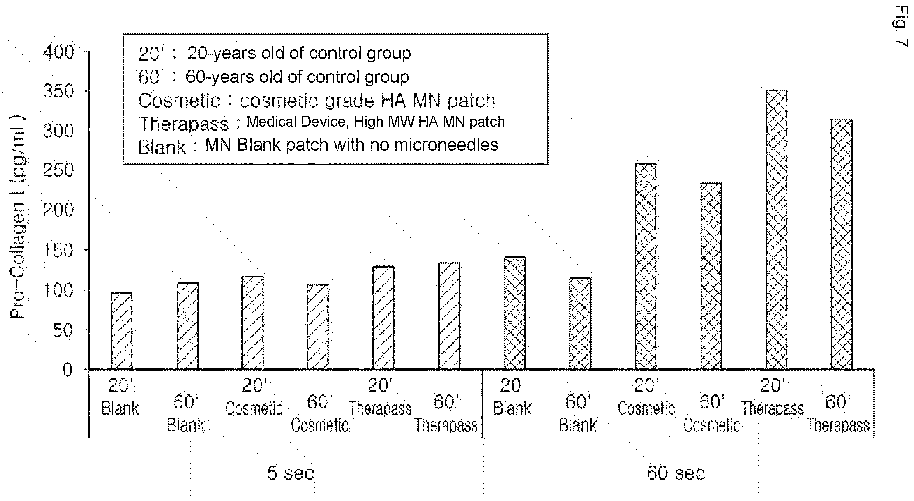

FIG. 7 is a chart showing experimental results obtained by detecting Pro-Collagen 1 after attaching three control groups to the skin of each of a 20-years old of patient group and a 60-years old of patient group and detaching the three control groups from the skins after a time lapse of 5 seconds and 60 seconds in a second embodiment of the present disclosure, wherein the first control group is a blank patch not provided with microneedles, the second control group is a microneedle patch formed of low-molecular-weight hyaluronic acid, and the third control group is a microneedle patch formed of high-molecular-weight hyaluronic acid.

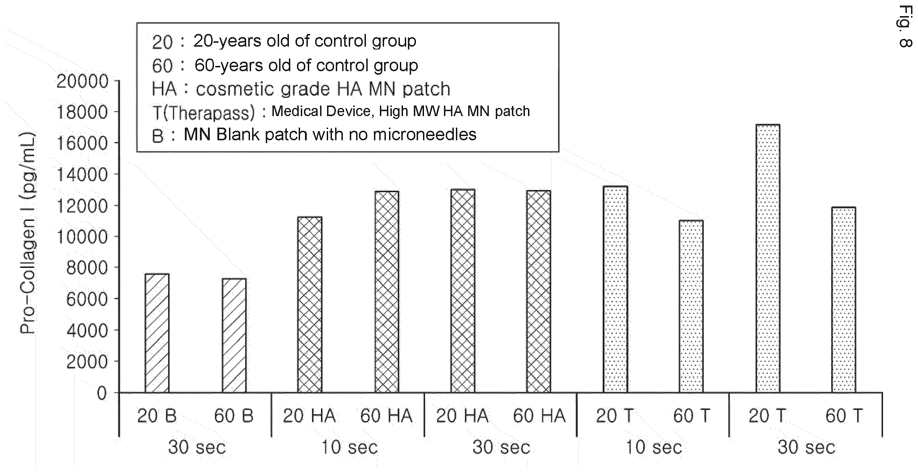

FIG. 8 is a chart showing results obtained by detecting Pro-Collagen 1 after attaching the three control groups to the skin of each of the 20-years old of patient group and the 60- years old of patient group and detaching the three control groups from the skin after a time lapse of 10 seconds and 30 seconds in the second embodiment of the present disclosure.

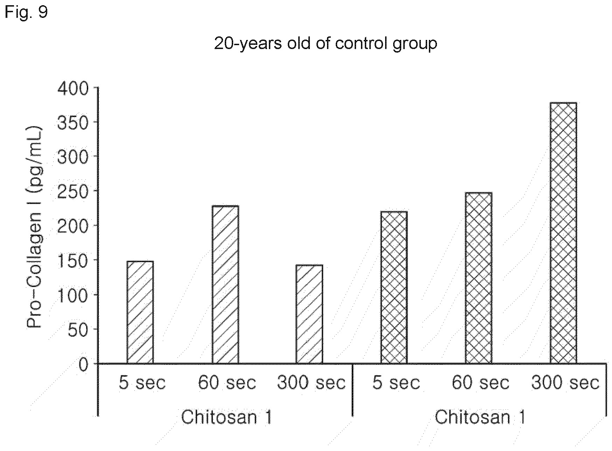

FIG. 9 is a chart showing experimental results obtained by detecting Pro-Collagen 1 after attaching two control groups to the skin and detaching the two control groups from the skin after a time lapse of 5 seconds, 60 seconds and 300 seconds in a third embodiment of the present disclosure, wherein the first control group is a microneedle patch formed of low-molecular-weight chitosan and the second control group is a microneedle patch formed of high-molecular-weight chitosan.

### DETAILED DESCRIPTION

In the following detailed description of the present disclosure, references are made to the accompanying drawings that show, by way of illustration, specific embodiments in which the present disclosure may be practiced. These embodiments are described in sufficient detail to enable those skilled in the art to practice the present disclosure. It is to be understood that the various embodiments of the present disclosure, although different from each other, are not necessarily mutually exclusive. For example, specific shapes, structures and characteristics described herein may be implemented as modified from one embodiment to another without departing from the spirit and scope of the present disclosure. Furthermore, it shall be understood that the positions or arrangements of individual elements within each of the embodiments may also be modified without departing from the spirit and scope of the present disclosure. Therefore, the following detailed description is not to be taken in a limiting sense, and the scope of the present disclosure is to be taken as encompassing the scope of the appended claims and all equivalents thereof.

Hereinafter, various preferred embodiments of the present disclosure will be described in detail with reference to the accompanying drawings to enable those skilled in the art to easily implement the present disclosure.

FIG. 2 is a conceptual view illustrating a skin biopsy method according to the present disclosure.

The upper left view in FIG. 2 shows a microneedle patch which is currently available as the product name "Therapass" in the market. This product is one sold by the present applicant and commercially available as a microneedle patch formed of high-molecular-weight hyaluronic acid having a molecular weight of 1,000 kDa in the marketplace. The lower left view in FIG. 2 shows a state in which such a microneedle patch is applied to the skin, more specifically, microneedles of the microneedle patch have penetrated to the dermal layer of the skin. In the right view in FIG. 2, there is conceptually shown a representative technical spirit of the present disclosure in which biopsy is performed using proteins in the skin adhering to microneedles of the microneedle patch when detaching the microneedle patch from the skin after being attached to the skin of a dermatitis patient. The term "Bio-Mining" used herein means extracting proteins in the skin of an object for biopsy.

FIG. 3 shows four control groups to be tested in a first embodiment of the present disclosure. In the four control groups, the first control group is a blank patch not provided with microneedles, the second group is a solid microneedle made of a metallic material, the third group is a microneedle patch formed of low-molecular-weight hyaluronic acid, and the fourth group is a microneedle patch formed of high-molecular-weight hyaluronic acid, wherein a molecular weight of the hyaluronic acid used in the microneedle patch formed of low-molecular-weight hyaluronic acid is 110 kDa, and a molecular weight of the hyaluronic acid used in the microneedle patch formed of high-molecular-weight hyaluronic acid is 1,000 kDa.

FIG. 4 is a chart showing results obtained by performing a Pro-Collagen 1 detection experiment with respect to each of a 30-years old of patient group and a 60-years old of patient group with the four control groups used in the first embodiment of the present disclosure.

On the x-axis in FIG. 4, the blank patch not provided with microneedles, the microneedle patch formed of low-molecular-weight hyaluronic acid (L.M. HA) and the microneedle patch formed of high-molecular-weight hyaluronic acid (H.M. HA) are indicated. On the y-axis in FIG. 4, a mass of the detected procollagen 1 per unit volume is indicated in terms of picogram (pg) per milliliter (mL). Among the four control groups, the second control group as the solid microneedle made of the metallic material is omitted herein. This is because quantification of the protein itself was in no consideration at all. The term "ELISA" indicated in FIG. 4 refers to a method of measuring the activity of an enzyme labeled on an antibody or antigen to quantitatively measure the strength and amount of the antigen-antibody reaction, which has been used in modem biotechnology. Such an ELISA method was employed throughout the experiments of the present disclosure.

A point to pay attention to in the chart of FIG. 4 is how the microneedle patch of the present disclosure is substantially different from the blank patch as the first control group, which is a conventional patch which has been used prior to the present disclosure. This is because if the experimental results of the microneedle patch of the present disclosure is not substantially different from those of the blank patch, the present disclosure has no meaning to contribute to the improvement of the effects of the biopsy. Referring to the results illustrated in FIG. 4, the amount of detected Pro-Collagen 1 in the microneedle patch formed of low-molecular-weight hyaluronic acid (having 110 kDa) was not substantially different from that of the blank patch. However, it can be seen that the amount of detected Pro-Collagen 1 in the microneedle patch formed of high-molecular-weight hyaluronic acid (having (1,000 Kpa) was significantly increased compared with those of the blank patch and the microneedle patch formed of low-molecular-weight hyaluronic acid.

Another experimental result is shown in FIG. 5. FIG. 5 is a chart showing results obtained by performing a Pro-Collagen 1 detection experiment with respect to each of a 30- years old of patient group and a 60-years old of patient group with the four control groups used in the first embodiment of the present disclosure, in which the y-axis represents absorbance OD. The chart of FIG. 5 is different from the chart of FIG. 4 in that the y-axis represents absorbance OD instead of the mass of Pro-Collagen 1 per unit volume. Based on a calibration curve using a standard substance, it is possible to perform quantitative estimation of the extracted biological tissue. Accordingly, by measuring a value of the absorbance OD in each group, it is possible to perform the quantitative estimation of Pro-Collagen 1. As may be seen from the results illustrated in FIG. 5, the value of the absorbance in the microneedle patch formed of low-molecular-weight hyaluronic acid was slightly distinct from that in the blank patch, but these values may be regraded to be substantially identical to each other. However, the absorbance in the microneedle patch formed of high-molecular-weight hyaluronic acid was significantly different from that in the blank patch.

The present inventors continuously conducted the experiments to analyze the reason why such a significant difference occurs according to the molecular weight of the hyaluronic acid, which is a component of the microneedle patch, and, as a result, found that the significant difference is caused by the dissolution rate of the hyaluronic acid into the skin according to the difference in molecular weight. The component of the commercially available microneedle patch is mainly a biocompatible and biodegradable polymer substance. The term "biocompatible substance" used herein refers to a substance that is not toxic to the body and is chemically inert. The term "biodegradable substance" used herein refers to a substance that can be decomposed by fluids, enzymes, or microorganisms in the body. In addition, it is known that the biodegradable substance tends to have a relatively high dissolution rate into the body as the molecular weight decreases, and a relatively low dissolution rate into the body as the molecular weight increases. Meanwhile, examples of the biocompatible polymer substance which is known in the art may be include substances as follows:

Hyaluronic acid (HA), gelatin, chitosan, collagen, alginic acid, pectin, carrageenan, chondroitin (sulfate), dextran (sulfate), polylysine, carboxymethyl titin, fibrin, agarose, pullulan, cellulose, polyvinylpyrrolidone (PVP), polyethylene glycol (PEG), polyvinyl alcohol (PVA), hydroxypropyl cellulose (HPC), hydroxyethyl cellulose (HEC), hydroxypropylmethyl cellulose (HPMC), sodium carboxymethyl cellulose, polyalcohol, arabic gum, alginate, cyclodextrin, dextrin, glucose, fructose, starch, trehalose, glucose, maltose, lactose, lactulose, fructose, turanose, melitose, melezitose, dextran, sorbitol, xylitol, palatinit, polylactic acid, polyglycolic acid, polyethylene oxide, polyacrylic acid, polyacrylamide, polymethacrylic acid , polymaleic acid, poly(ethyleneglycol)/polyester, chitosan/glycerol phosphate, polyphosphazene, polycaprolactone, polycarbonate, poly(ethylene glycol)poly(propylene glycol), polycyanoacrylate, polyorthoester, poly(N-(2-hydroxyethyl)methacrylamide-lactate, poly(propylene phosphate), and the like.

When the microneedle patch is attached to the skin, proteins may adhere to the structure of the biocompatible microneedles in the course of entering the microneedles of the microneedle patch to the dermal layer of the skin, and while maintaining a state after the entry. Thus, the proteins may be extracted. However, since the microneedles of the microneedle patch formed of low-molecular-weight hyaluronic acid, to which bio-proteins have been adhered, are dissolved at a relatively high dissolution rate, when the microneedle patch is attached to the skin for a long period of time, the bio-proteins adhering to the skin may be lost due to the dissolution of the microneedles itself. For this reason, the experimental results as illustrated in FIG. 4 was obtained. The supporting results are shown in FIG. 6.

FIG. 6 is a chart showing results obtained by measuring changes in length before and after performing skin biopsy using the microneedle patch formed of low-molecular-weight hyaluronic acid and the microneedle patch formed of high-molecular-weight hyaluronic acid in the four control groups used in the first embodiment of the present disclosure.

As may be seen from the results illustrated in FIG. 6, the microneedles of the microneedle patch formed of low-molecular-weight hyaluronic acid of 110 kDa shown a reduction in length by a level of about 30% while being attached to the skin of the body. Meanwhile, the microneedles of the microneedle patch formed of high-molecular-weight hyaluronic acid of 1,000 kDa shown a reduction in length by a level of about 10% while being attached to the skin. This was analyzed that, since the microneedles of the microneedle patch formed of low-molecular-weight hyaluronic acid, to which bio-proteins have been adhered, are dissolved at a relatively fast dissolution rate, when the microneedle patch is attached to the skin for a long period of time, the bio-proteins adhering to the skin may be lost due to the dissolution of the microneedles itself. In the experiments of the first embodiment shown in FIGS. 4 to 6, the attachment time of each control group to the skin was 5 minutes. Accordingly, the present inventors found needs to conduct experiments for different periods of times while making the attachment time of each control group to the skin lower than 5 minutes, and consequently designed experiments according to a second embodiment.

FIG. 7 is a chart showing experimental results obtained by detecting Pro-Collagen 1 after attaching three control groups to the skin of each of a 20-years old of patient group and a 60-years old of patient group and detaching the three control groups from the skins after a time lapse of 5 seconds and 60 seconds in a second embodiment of the present disclosure, wherein the first control group is a blank patch not provided with microneedles, the second control group is a microneedle patch formed of low-molecular-weight hyaluronic acid, and the third control group is a microneedle patch formed of high-molecular-weight hyaluronic acid. The molecular weight of the low-molecular-weight hyaluronic acid and the molecular weight of the high-molecular-weight hyaluronic acid are the same as those in the experiment in the first embodiment. As the microneedle patch formed of low-molecular-weight hyaluronic acid, a cosmetic microneedle patch which is commercially available from the present applicant was used, and as the microneedle patch formed of high-molecular-weight hyaluronic acid, a medical microneedle patch (product name: Therapass) which is commercially available from the present applicant was used. As a result of the experiment in the first embodiment, the solid microneedle made of a metallic material was not used as the control group in the present embodiment because it was significantly poor in the bioprotein extraction performance.

The results of the experiments illustrated in FIG. 7 show the following facts. First, there was no significant difference between the microneedle patch and the blank patch at the attachment time of 5 seconds. Second, at the attachment time of 60 seconds, the microneedle patches (formed of low-molecular-weight hyaluronic acid and high-molecular-weight hyaluronic acid) shown a significantly superior bio-protein extraction performance compared with the blank patch. Third, even at the attachment time of 60 seconds, the microneedle patch formed of high-molecular-weight hyaluronic acid was better in bio-protein extraction performance than the microneedle patch formed of low-molecular-weight hyaluronic acid. Based on the experimental results illustrated in FIG. 7, the present inventors further conducted experiments illustrated in FIG. 8 to obtain additional results when the attachment time exceeds 5 seconds and is shorter than 60 seconds.

FIG. 8 is a chart showing results obtained by detecting Pro-Collagen 1 after attaching the three control groups to the skin of each of a 20-years old of patient group and a 60-years old of patient group and detaching the three control groups from the skin after a time lapse of 10 seconds and 30 seconds in the second embodiment of the present disclosure.

In the case of the blank patch, there was no significant change at the attachment time of 10 seconds or 30 seconds. In the chart of FIG. 8, only the experimental results obtained at the attachment time of 30 seconds are shown. The results illustrated in FIG. 8 show the following facts. First, it was possible to confirm that the bio-protein extraction performance of the microneedle patches (formed of low-molecular-weight hyaluronic acid and high-molecular-weight hyaluronic acid) was significantly improved compared to that of the blank patch even at the attachment time of 10 seconds. Second, at the attachment time of 10 seconds, the bio-protein extraction performances of the microneedle patch formed of low-molecular-weight hyaluronic acid and the microneedle patch formed of high-molecular-weight hyaluronic acid were substantially identical to each other. Third, at the attachment time of 30 seconds, the bio-protein extraction performance of the microneedle patch formed of high-molecular-weight hyaluronic acid was significantly improved compared with the microneedle patch formed of low-molecular-weight hyaluronic acid.

The experiment illustrated in FIG. 8 is evaluated to have technical significance in that for the attachment time of 10 seconds or more, the biopsy performance of the microneedle patch was significant, and for the attachment time of about 10 seconds for which the microneedles of the microneedle patch formed of low-molecular-weight hyaluronic acid may not be dissolved into the skin, the microneedle patch formed of low-molecular-weight hyaluronic acid and the microneedle patch formed of high-molecular-weight hyaluronic acid were substantially identical to each other in the biopsy performance.

Finally, the present inventors conducted the experiment illustrated in FIG. 9 using a microneedle patch formed of a chitosan material to determine whether a microneedle patch formed of a biocompatible polymer substance other than the hyaluronic acid is suitable for use in biopsy. Chitosan is a biocompatible polymer substance, like hyaluronic acid, but is not biodegradable. Chitosan is a substance that is processed to allow chitin contained in the crustacean to be easily absorbed into the body, and has been known to have an effect of activating aged cells to inhibit aging and enhance immunity. Chitosan, which has been proven effective in terms of skin cell activation, is widely used as a cosmetic ingredient and is also used as a component of a microneedle patch for skin care purposes.

FIG. 9 is a chart showing experimental results obtained by detecting Pro-Collagen 1 after attaching two control groups to the skin and detaching the two control groups from the skin after a time lapse of 5 seconds, 60 seconds and 300 seconds in a third embodiment of the present disclosure, wherein the first control group is a microneedle patch formed of low-molecular-weight chitosan and the second control group is a microneedle patch formed of high-molecular-weight chitosan.

In the experiment illustrated in FIG. 9, the molecular weight of the low-molecular-weight chitosan 1 was 50 to 100 kDa. The molecular weight of the high-molecular-weight chitosan 2 was 250 to 350 kDa. After the microneedle patch is attached to the skin of a 20years old of patient group for 5 seconds, 1 minutes, and 5 minutes, Pro-Collagen 1 was detected. From the results of the experiment in the third embodiment illustrated in FIG. 9, the present inventors confirmed that chitosan which is one of biocompatible polymer substances has a bio-protein extraction function for biopsy, like the hyaluronic acid. It should be noted that various biocompatible polymer substances other than the hyaluronic acid and the chitosan described herein may be candidates for a material of a microneedle patch for use in a minimally-invasive skin biopsy.

While the present disclosure has been described in the foregoing by way of embodiments and drawings which are defined with specific matters such as specific components and the like, this is only the one provided to aid in a more general understanding of the present disclosure, and the present disclosure is not limited to the above embodiments, and various modifications and variations can be made from the substrate to those skilled in the art to which the present disclosure pertains.

Accordingly, the spirit of the present disclosure should not be defined as limited to the embodiments described above, and all that have been equivalently or equivalently modified with the claims to be described below, as well as those to be within the scope of the spirit of the present disclosure.

## Claims

1. A minimally-invasive skin biopsy method, the method comprising: preparing a microneedle patch;
attaching the microneedle patch to a skin of an object;
maintaining a state in which the microneedle patch is attached to the skin of the object for a predetermined period of time; and
detaching the microneedle patch from the skin of the object after the predetermined period of time, and analyzing a protein component of the object, which adheres to a microneedle of the microneedle patch,
wherein the microneedle is made of a biocompatible polymer substance.

2. The minimally-invasive skin biopsy method of Claim 1, wherein the biocompatible polymer substance, which is a material of the microneedle, is a hyaluronic acid or a chitosan.

3. The minimally-invasive skin biopsy method of Claim 2, wherein when the material of the microneedle is the hyaluronic acid, the predetermined period of time is determined in advance in consideration of a dissolution rate according to a molecular weight of the hyaluronic acid.

4. The minimally-invasive skin biopsy method of Claim 3, wherein, as the molecular weight of the hyaluronic acid increases, the predetermined period of time for which the microneedle patch is attached to the skin of the object is increased to improve a biopsy performance.
